# FASCICULE DE BREVET EUROPEEN

(11) **EP 2 673 046 B1**
(45) Date de publication et mention de la délivrance du brevet: **30.05.2018**
(21) Numéro de dépôt: 12717146.0
(22) Date de dépôt: 09.02.2012
(51) Int. Cl.: A61N 5/10, A61B 6/08

(54) **PROCÉDÉ DE RÉGLAGE ET DE POSITIONNEMENT À L'AIDE DE SYSTÈMES LASERS ET SON DISPOSITIF DE MISE EN OEUVRE**
STEUERUNGS- UND POSITIONIERUNGSVERFAHREN MIT HILFE VON LASERSYSTEMEN UND VORRICHTUNG ZUR DURCHFÜHRUNG DIESES VERFAHRENS
METHOD OF CONTROL AND OF POSITIONING WITH THE AID OF LASER SYSTEMS, AND DEVICE FOR IMPLEMENTING SAID METHOD

(30) Priorité: 09.02.2011 BE 201100082
(43) Date de publication de la demande: 18.12.2013
(73) Titulaire: Cyrpa International, 1180 Bruxelles (BE)
(72) Inventeur: BOULINIERE, Pascal, 77410 Villeroy (FR)
(74) Mandataire: Petsis, Christos
(86) Numéro de dépôt international: PCT/BE2012/000007
(87) Numéro de publication internationale: WO 2012/106782

(56) Documents cités:
- EP-A1- 0 753 285
- EP-A1- 1 854 412
- GB-A- 2 443 432
- US-A- 4 123 660
- US-A1- 2011 001 987

## Description

### Domaine de l'invention

L'invention se rapporte à un procédé de réglage et de positionnement à l'aide de systèmes lasers, essentiellement dans le secteur de la radiothérapie.

### Arrière-plan technologique

De nos jours, plus de 50 % des patients souffrant d'un cancer reçoivent un traitement par radiothérapie. Ce traitement peut suffire en certains cas. Il se peut cependant que l'équipe médicale chargée du suivi du patient estime qu'une ablation chirurgicale de la tumeur doit suivre le traitement de radiothérapie.

Depuis près d'un siècle, la radiothérapie fait partie de l'arsenal thérapeutique des maladies cancéreuses. Après quelques balbutiements, cette technique est bien maîtrisée aujourd'hui et constitue, avec la chimiothérapie et la chirurgie, le traitement le plus répandu des cancers, aboutissant à un grand nombre de guérisons.

Le principe de la radiothérapie est simple : il s'agit d'exposer les cellules cancéreuses à une ionisation, c'est-à-dire une émission de radiations, qui va altérer la composition de l'information génétique des cellules cancéreuses. De nos jours, les spécialistes ont à leur disposition un éventail très large de qualité et de quantité de radiations ionisantes.

Ce matériel génétique contenu sous la forme d'ADN subit des transformations qui rendront la cellule irradiée incapable de se reproduire. Cette sorte de stérilisation réduit ainsi la reproduction anarchique de ces cellules malignes, responsables du cancer. Certes, les cellules normales peuvent également être affectées par ces radiations, mais leurs taux de réparation est supérieur à ceux des cellules cancéreuses, ce qui produit ainsi un effet dit différentiel. Cet effet différentiel explique le bénéfice de la radiothérapie pour les malades. Ainsi, la mission du radiothérapeute est de réussir à tuer les cellules cancéreuses et à préserver les cellules saines du patient.

La radiothérapie peut ainsi être prescrite comme traitement curatif, à savoir pour détruire la tumeur, comme traitement palliatif pour atténuer la douleur, ou encore comme traitement adjuvant pour préparer ou pour compléter une intervention chirurgicale ou une chimiothérapie.

Avant que le traitement proprement dit ne puisse être suivi, la radiothérapie doit être préparée. Il convient, à cet égard, de déterminer la localisation et la dose de radiation qui va être administrée au patient. Dans une première étape, les soins sont préparés grâce à un scanner, qui permet de fixer les limites de la zone devant être irradiée. Une reconstruction d'images logicielle en trois dimensions permettra une localisation très exacte de la tumeur. Le patient devra rester immobile, afin que la localisation de la zone soit la plus précise possible.

Une fois que la zone à traiter est déterminée, le praticien applique sur le patient des petits points de tatouage ou de la peinture, notamment « fuchsine », pour identifier la zone à traiter. Cette première étape prend de 30 minutes à une heure. C'est à ce stade qu'intervient l'utilisation des lasers de positionnement.

La deuxième étape consiste à analyser toutes les données acquises par la machine pour que le radiothérapeute et le radiophysicien déterminent l'appareil de soin à utiliser, la répartition de la dose, la taille, le nombre et l'orientation des champs d'irradiation, qui sont les plus appropriés. La quantité de radiations prescrites dépend de l'âge et de l'état de santé du patient, ainsi que de la localisation et du type de cancer.

La dose totale est alors fractionnée en plus faibles doses, car l'effet est cumulatif. Les doses fractionnées seront dès lors administrées en diverses séances, à chacune de celles-ci. Ces séances seront étalées en moyenne sur deux à sept semaines.

Les lasers de positionnements équipent les salles de diagnostic - étape 1-, d'une part, et les salles de traitement de radiothérapie - étape 2 -, d'autre part. Ils peuvent être de deux types différents.

Dans les salles de diagnostic, ils servent à guider les manipulateurs pour permettre à ceux-ci d'apposer le marquage sur la peau des patients : c'est la première étape du traitement.

Dans les salles de traitement, ils servent à positionner le patient dans l'accélérateur : c'est la dernière étape du traitement.

Les salles de diagnostic abritent également la simulation virtuelle. C'est dans ces salles que la tumeur sera parfaitement identifiée et localisée grâce à une machine adaptée, le scanner.

Une installation standard de type connu est constituée de 5 lasers mobiles placés de part et d'autre de la table du scanner qui vont générer 5 nappes lasers, à concurrence d'une nappe par laser. Les cinq lasers sont pilotés par un système informatique présent dans la salle de contrôle.

Les lasers forment trois plans distincts, à savoir un plan horizontal, un plan vertical sensiblement transversal, ces deux plans formant une croix, et un autre plan vertical encore qui est généralement perpendiculaire dans l'axe de la table, axe dit sagittal.

Les nappes verticales précitées doivent être confondues en un plan unique. Ce plan doit être parfaitement vertical et parallèle au plan vertical de l'isocentre machine. Les nappes horizontales doivent être confondues en un plan unique. Ce plan doit être parfaitement horizontal et perpendiculaire au plan vertical de l'isocentre machine. La nappe sagittale doit être parfaitement verticale et perpendiculaire au plan vertical de l'isocentre machine.

### Etat de la technique

Il existe à cet égard des systèmes de réglage des lasers connus, comme décrit dans le document français FR-2770763-A1, lesquels ne présentent toutefois aucun moyen de mesure extérieur, ce qui se traduit par une absence de contrôle s'avérant pourtant importante, voire essentielle.

Il existe également un autre dispositif plus récent consistant en un système de surveillance du positionnement de laser sur un appareil du diagnostic et/ou de thérapie tel que décrit dans DE-10200602168-A1 divulguant un dispositif comportant un appareil dit LAP qui est composé de trois éléments distincts : un premier cube permettant la visualisation de l'isocentre du scanner après acquisition d'images, un second cube comportant les capteurs d'acquisition des positions des nappes laser et un support recevant les deux cubes. La méthodologie décrite consiste à poser le support sur la table du scanner et ensuite de mettre en place le cube. Une séance d'acquisition d'images est alors déclenchée, et après plusieurs manipulations du support, la position définitive est validée, position où le support est parfaitement perpendiculaire au plan isocentre du scanner. Le premier cube est alors retiré et le second cube est mis en place sur le support. Les lasers externes sont alors allumés et leur position est mesurée par les capteurs linéaires placés sur le second cube.

Le document EP 1854412-A1 divulgue un dispositif qui est constitué de 3 blocs distincts dans lequel le premier bloc sert de support aux deux autres. Sur ce support est placé le premier dispositif 38 qui permet d'ajuster l'ensemble par rapport à l'isocentre de la machine. Une fois cet ajustement effectué, le second bloc 22 est mis en place. Sur ce second bloc, on retrouve des capteurs au nombre de 16, qui analysent la position des nappes lasers. Celles-ci sont ensuite ajustées mais il n'y est pas stipulé comment. Certes, ce document divulgue l'acquisition d'images permettant de contrôler la position d'un dispositif grâce à la présence d'une bille de centrage, ainsi que la position des nappes lasers étant déterminée grâce aux cellules ou capteurs embarquées dans un autre appareil, et encore l'ajustage de chaque nappe laser. De plus, ce document divulgue aussi deux dispositifs bien distincts, à savoir un dispositif avec une bille de centrage et un autre dispositif avec des capteurs de position, ce qui nécessite le changement de dispositif et ainsi plusieurs manipulations des opérateurs. Ceci alourdit dès lors la procédure de manipulation, ce qui pose problème dans ce type d'intervention.

Ainsi à ce jour, il existe sur le marché des systèmes de positionnement laser comparables, dont la technologie proposée est proche de celle proposée ci-après. Ces systèmes se composent tous de têtes optiques, qui génèrent les nappes laser précitées et qui sont montées sur des rails plus ou moins performants. La couleur des nappes lasers est fixée une fois pour toutes à l'installation des systèmes chez l'utilisateur. Un outil spécifique est alors utilisé pour le réglage du système, le positionnement des lasers. Cet outil est généralement utilisé seulement par le personnel du fournisseur du système. La précision finale du positionnement est de l'ordre du millimètre. Il en ressort que le réglage des systèmes actuels est très délicat et nécessite souvent un personnel spécialisé, ce qui peut nuire à la flexibilité de l'ensemble, ainsi d'ailleurs qu'à son coût d'exploitation. De plus, la conception des rails utilisés et le principe de réglage du système ne permettent de garantir ni la répétabilité, ni la fiabilité des mesures. Par ailleurs, il n'existe aucune information permettant de s'assurer que les nappes lasers soient véritablement à la position demandée par l'opérateur.

A cela s'ajoute que la couleur des nappes est quelquefois gênante pour le repérage sur certaines couleurs de peau. Enfin, le logiciel de pilotage associé est parfois extrêmement restreint et très éloigné des standards de l'assurance Qualité en milieu hospitalier.

Le brevet américain US 4 123 660 du 31 octobre 1978 divulgue quant à lui un appareil optique qui permet de vérifier l'alignement des lasers dans une salle d'accélérateur, qui est sans incidence toutefois avec le système automatique visé ici.

Par ailleurs, le document US 2011/001987 du 6 janvier 2011 divulgue un appareil générant une croix à partir d'une diode source laser sans rapport avec le dispositif visé selon la présente invention.

Il en est de même avec le document GB 24 43 432 A du 31 octobre 2006 divulguant un appareil de guidage portatif d'objet à l'aide de rayons sans rapport avec le dispositif visé. Quant au document EP 0 753 285 A1, il se rapporte à des lasers de positionnement certes, mais ce document ne fait pas mention d'un appareil de réglage des nappes laser.

Tous ces éléments introduisent une restriction quant à la qualité qui nuit à l'efficacité finale du traitement du patient, ce qui constitue dès lors un inconvénient important qu'il convient de réduire.

### Objet de l'invention

La présente invention a pour but de remédier à cette absence parmi les dispositifs connus.

### Exposé de l'invention

A cette fin, il est proposé selon l'invention un procédé d'utilisation d'un dispostif tel que défini dans la revendication principale 1 consistant en une mise en place du dispositif sur la table du scanner accompagné d'un préréglage de la position grâce aux trois pieds supports et aux niveaux à bulles embarqués, d'un déplacement de la table du scanner pour mise en place de la face avant du dispositif dans le plan de l'isocentre du scanner, et d'un positionnement grâce aux lasers internes du scanner, ainsi que d'une acquisition d'images. Les images obtenues permettent de contrôler la bonne position du dispositif grâce aux diverses billes de centrage présentes sur la face avant.

Ensuite a lieu la finalisation de la position du dispositif. Une fois la position validée, la table du scanner est déplacée de la distance nominale entre l'isocentre machine et l'isocentre laser. Cette distance est fixe. Elle est déterminée et validée lors de l'installation des lasers. Le dispositif est raccordé à un Tablet PC gérant les positions des lasers et il est mis sous tension, avec mise sous tension des lasers avec remise à zéro des positions.

Le démarrage de la séquence de recherche des nappes lasers a lieu par multiplexage. La position de chaque laser est alors déterminée grâce aux capteurs de position embarquées dans le dispositif. L'acquisition des valeurs se fait et le transfert vers le Tablet PC de la phase d'ajustage de chaque nappe laser.

Sa grande spécificité est, en effet, étroitement liée à la nature particulière du dispositif de mise en oeuvre du procédé, et requiert du coup une définition précise de chacune des étapes d'utilisation du dispositif selon l'invention.

La présente invention se rapporte également au dispositif d'utilisation, respectivement de mise en oeuvre du procédé tel que défini dans la revendication principale de dispositif. Ainsi, le dispositif selon la présente invention est composé d'un ensemble unique composé de sept pièces distinctes et assemblé de façon indémontable. Il embarque à la fois les 12 capteurs de position et une carte électronique de gestion. Sa conception est unique, et ce principalement par le fait qu'il est composé d'un seul bloc au lieu de trois, voire plusieurs. Cette architecture évite ainsi plusieurs manipulations des opérateurs, ce qui réduit sensiblement le risque d'erreurs humaines du même coup et fiabilise le résultat final.

Suivant un mode de réalisation additionnel du dispositif selon l'invention baptisé tête optique, il comprend deux éléments laser générant une nappe de couleur rouge ou verte, au choix de l'opérateur. Grâce à cette mesure, on obtient un composant fiable et stable.

L'assemblage des deux caractéristiques dans un seul dispositif évite le changement du dispositif et évite ainsi plusieurs manipulations des opérateurs grâce au procédé, resp. dispositif visé selon la présente invention. Cette solution doit ainsi être considérée comme remarquable, d'autant qu'il propose le déplacement de la table du scanner de la distance nominale entre l'isocentre machine et l'isocentre laser.

Suivant un mode de réalisation avantageux du dispositif selon l'invention, il incorpore deux éléments lasers standards industriels. Le dispositif est équipé sur sa base de trois pieds réglables permettant un réglage en azimut sur la table du scanner. Des niveaux à bulles sont également disposés sur la plaque de base du dispositif pour aider au positionnement par les opérateurs.

Suivant un autre mode de réalisation encore avantageux du dispositif selon l'invention, le dispositif est muni de moyens d'articulation, tels que charnières, permettant de le plier pour pouvoir être transporté plus aisément. En effet, il s'agit d'un dispositif portable, lui conférant ainsi une commodité d'utilisation. De plus, il l'est d'autant plus portable, qu'il est constitué d'un seul bloc au lieu de plusieurs.

Suivant un mode de réalisation préféré du dispositif selon l'invention, il est dédié au réglage automatique du système avec cellules de mesure intégrées et dimension calculée pour la meilleure précision. Cette particularité selon l'invention offre l'avantage d'une mesure précise de la position de chaque nappe et réglage de chaque nappe l'une part rapport à l'autre, ainsi qu'un réglage global et automatique du système en boucle fermée à partir d'un logiciel de pilotage.

Ainsi, grâce à la solution apportée par l'invention, tout le système selon l'invention est basé sur l'obtention d'un résultat de positionnement garanti, à savoir une précision finale considérablement renforcée qui est encore meilleure que 0,1 mm.

Le système proposé est fiable en diminuant significativement le nombre d'interventions de la part de l'installateur pour cause de panne ou de dysfonctionnement.

Le système selon l'invention procure une disponibilité significativement supérieure des salles équipées avec ce système.

Ce dernier offre une couleur de nappe laser qui est laissée au choix de l'opérateur, et qui est interchangeable instantanément.

Le système permet un réglage automatique de celui-ci, par l'utilisation d'un outil dédié, et ce avec une rapidité extrême, en quelques secondes.

Un autre avantage offert selon l'invention est une mesure de position de chaque nappe laser qui est d'une précision extrême, au centième.

Un autre avantage encore consiste en une répétabilité et une stabilité dans le temps du système qui est optimale. Suivant un mode de réalisation, le procédé utilise un logiciel pour la mise en oeuvre d'au moins une partie des étapes resp. phases du procédé. En particulier, il est proposé un logiciel dédié et adapté aux contraintes d'une utilisation en milieu hospitalier.

Ainsi, l'invention apporte une réponse adaptée pour obtenir un résultat optimal en termes de traitement et de sécurité pour le patient, en respectant toutes les recommandations de base des systèmes Qualité.

Suivant un mode de réalisation additionnel du dispositif selon l'invention, il est prévu une tête optique mécanisée avec des éléments mécaniques simples mais parfaitement calibrés et étudiés pour garantir tous les degrés de liberté nécessaire avec le maximum de précision. Grâce à cette mesure supplémentaire, on obtient une bonne fiabilité et répétabilité du système optique, ainsi qu'une stabilité temporelle et en température, et encore un filtrage des perturbations vibratoires externes.

Suivant un mode de réalisation préférentiel du dispositif selon l'invention, il est prévu un rail pour les mouvements de la tête optique avec roulements mécaniques de précision intégrant une mesure de position de la tête optique sur son rail. Grâce à cette autre mesure, on obtient une fiabilité améliorée et une précision des mouvements du dispositif optique, ainsi qu'une vérification de la position exacte des nappes laser en fonction des consignes données par l'opérateur.

D'autres propriétés et particularités du procédé et du dispositif selon l'invention sont définies dans les autres sous-revendications respectives.

D'autres détails et avantages sont précisés dans la description d'un mode de réalisation préféré de l'invention donnée ci-après, qui est illustré à l'aide des dessins annexés, fournis à titre d'exemple de réalisation non limitatif.

### Brève description des dessins

La figure 1 est une représentation schématique du montage d'un mode de réalisation du dispositif de base suivant l'invention.
La figure 2 est une représentation schématique d'un agencement de l'installation pour le balayage d'un patient permettant de fournir une vue droite du patient dans le système selon l'invention.
La figure 3 est une représentation schématique d'un agencement de l'installation pour le balayage d'un patient permettant de fournir une vue gauche du patient dans le système selon l'invention.
La figure 4 est une représentation schématique d'un agencement de l'installation pour le balayage d'un patient permettant de fournir une vue dite sagittale du patient dans le système selon l'invention.
La figure 5 représente une vue analogue aux dernières figures précédentes d'une variante préférée du dispositif destiné à être utilisé en association avec le système selon l'invention.
La figure 6 est une représentation schématique d'un agencement de l'installation pour le balayage d'un patient permettant de fournir une vue générale en perspective, à l'état déplié prêt à l'emploi du patient dans le système selon l'invention.
La figure 7 est une représentation schématique d'un agencement de l'installation pour le balayage d'un patient permettant de fournir une vue générale en perspective, à l'état replié, dans le système selon l'invention.
La figure 8 représente une vue de dessus d'un dispositif à tête optique prévu pour être utilisé en association avec le système précité de base selon l'invention.
La figure 9 est une représentation schématique d'un agencement particulier de l'invention.
La figure 10 est une représentation simplifiée d'un élément particulier de l'invention.
La figure 11 est une représentation simplifiée en schéma-bloc représentant des modules constituant les fonctionnalités du logiciel utilisé dans l'agencement de l'invention.

### Description

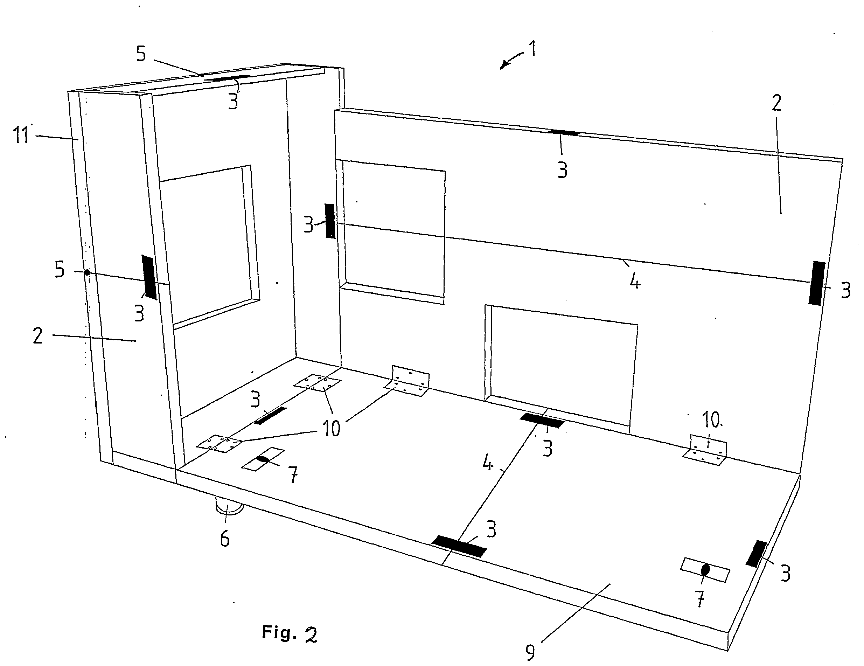

De façon générale, l'invention porte sur un dispositif dit intelligent, qui est constitué d'une plaque de base 9 et de plusieurs montants verticaux 2 en matière plastique, comme montré sur la figure 2. L'appareil supporte un certain nombre de photocellules linéaires 3 de détection des nappes laser 4, de préférence autour de douze, un certain nombre de billes à haute densité de repérage 5, de préférence autour de cinq, trois pieds supports 6 et deux niveaux à bulle 7.

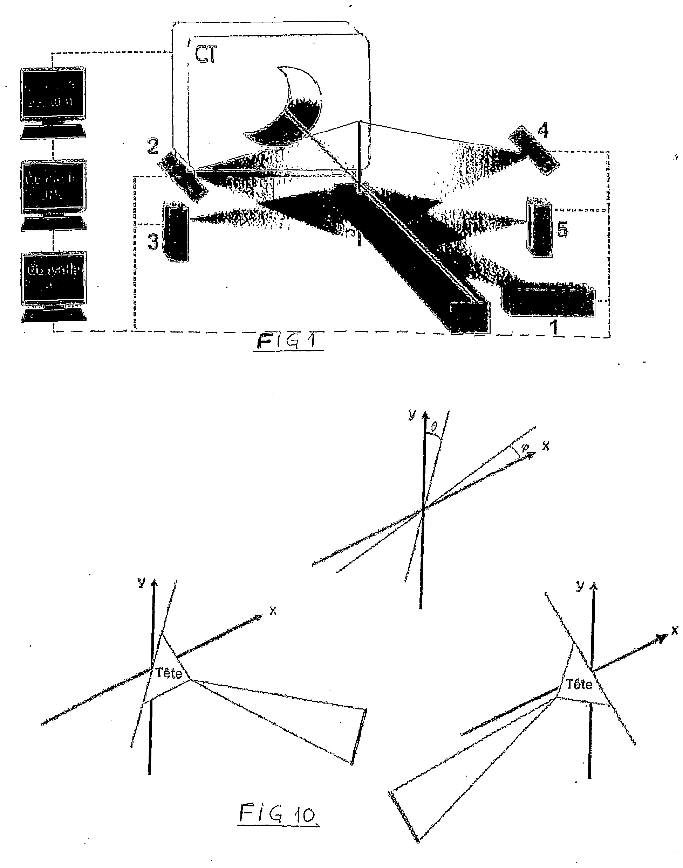

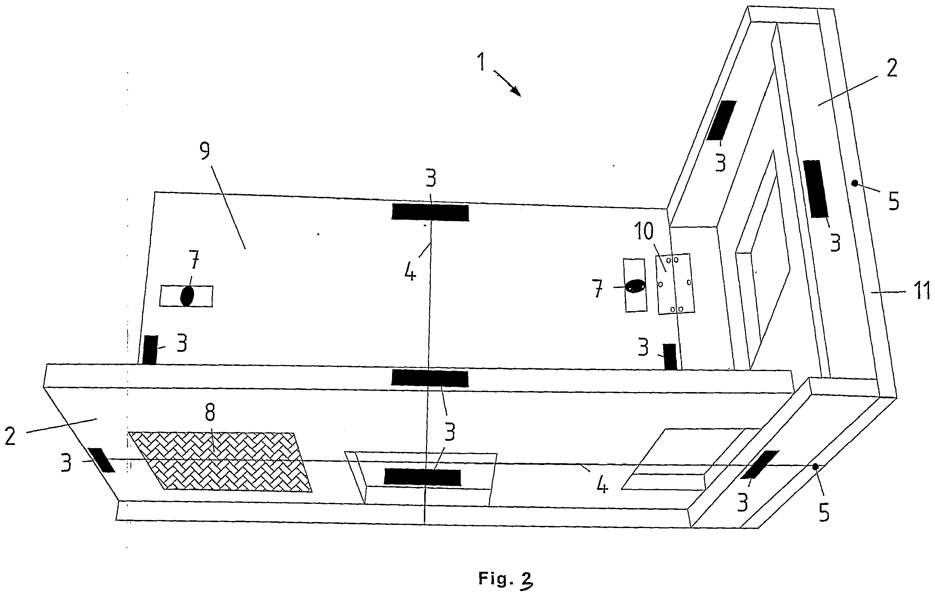

Chaque nappe laser 4 se projette sur trois cellules 3 disposées dans un volume. La position et l'angle exact de chaque nappe 4 sont ainsi déterminés. Ces informations sont enregistrées par une carte électronique de gestion 8 disposée dans l'appareil 1 et montrée sur la figure 3. Les informations sont ensuite transmises à un ordinateur qui effectue les corrections de positionnement en temps réel pour chaque nappe laser 4 comme montré sur la figure 1.

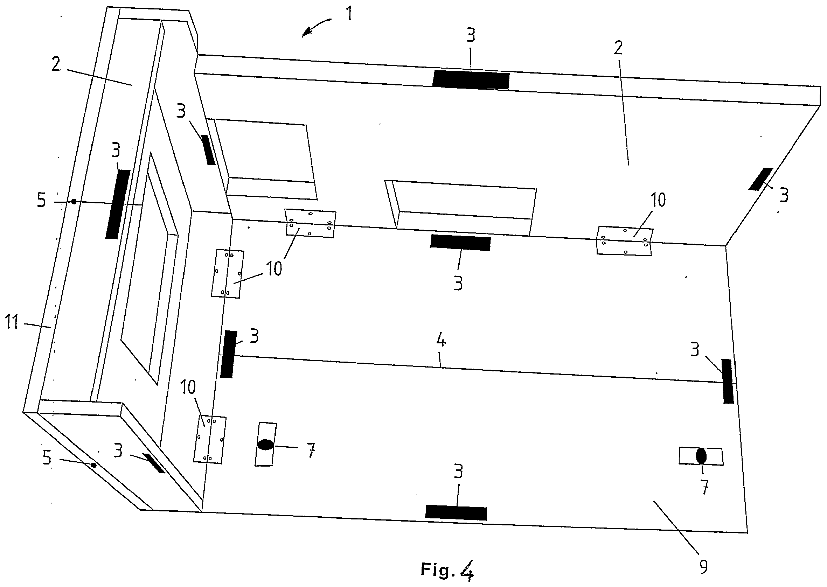

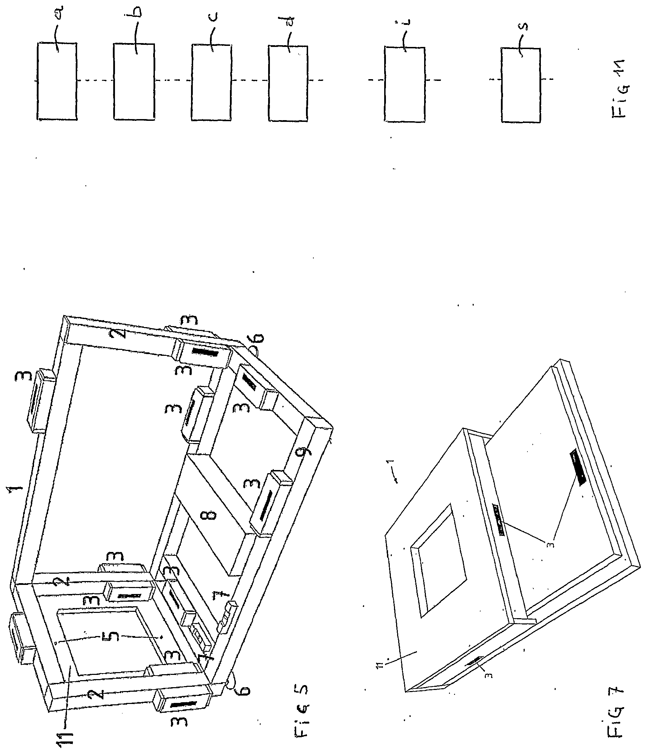

La figure 4 montre l'appareil 1 muni de charnières 10 permettant de le plier pour pouvoir être transporté plus aisément. En effet, la figure 7 montre qu'il s'agit d'un appareil portable, lui conférant ainsi une commodité d'utilisation.

La figure 5 montre l'appareil 1 équipé sur sa base desdits pieds qui sont réglables 6 permettant un réglage en azimut sur la table du scanner. Les niveaux à bulles précités 7 sont disposés sur la plaque de base 9 pour aider au positionnement par les opérateurs.

La procédure d'utilisation du dispositif de base est la suivante :
Mise en place de l'appareil sur la table du scanner ;
Pré-réglage de la position grâce aux trois pieds supports 6 et aux niveaux à bulle 7 embarqués ;
Déplacement de la table du scanner pour mise en place de la face avant 11 de l'appareil dans le plan de l'isocentre du scanner. Positionnement grâce aux lasers internes du scanner. Acquisition d'images. Les images obtenues permettent de contrôler la bonne position de l'appareil 1 grâce aux 5 billes de centrage 5 présentes sur la face avant 11. Finalisation de la position de l'appareil 1 ;
Une fois la position validée, déplacement de la table du scanner de la distance nominale entre l'Isocentre machine et l'Isocentre laser. Cette distance est fixe. Elle est déterminée et validée lors de l'installation des lasers ;
Raccordement de l'appareil 1 avec le Tablet PC gérant les positions des lasers ; Mise sous tension de l'appareil 1 ;
Mise sous tensions des nappes lasers 4 avec remise à zéro des positions ;
Démarrage de la séquence de recherche des nappes lasers 4 par multiplexage. La position de chaque nappe laser 4 est alors déterminée grâce aux cellules 3 embarquées sur le dispositif 1 ;
Acquisition des valeurs et transfert vers le Tablet PC ;
Démarrage par le Tablet PC de la phase d'ajustage de chaque nappe laser 4.

La figure 5 montre le plan général du dispositif 1 et notamment le volume qu'il délimite. Les photocellules 3 sont représentées. Le réglage d'une nappe 4 s'obtient à partir de 3 photocellules 3. Les photocellules 3 sont placées judicieusement et de façon ultra-précise sur le dispositif 1 de façon à ce qu'une fois réglées, les nappes 4 convergent toute au même point de l'espace appelé isocentre dispositif. Les billes de repérage 5 servent à obtenir une correspondance ultra-précise entre l'isocentre du dispositif et l'isocentre du scanner lors de la procédure de calibration du système.

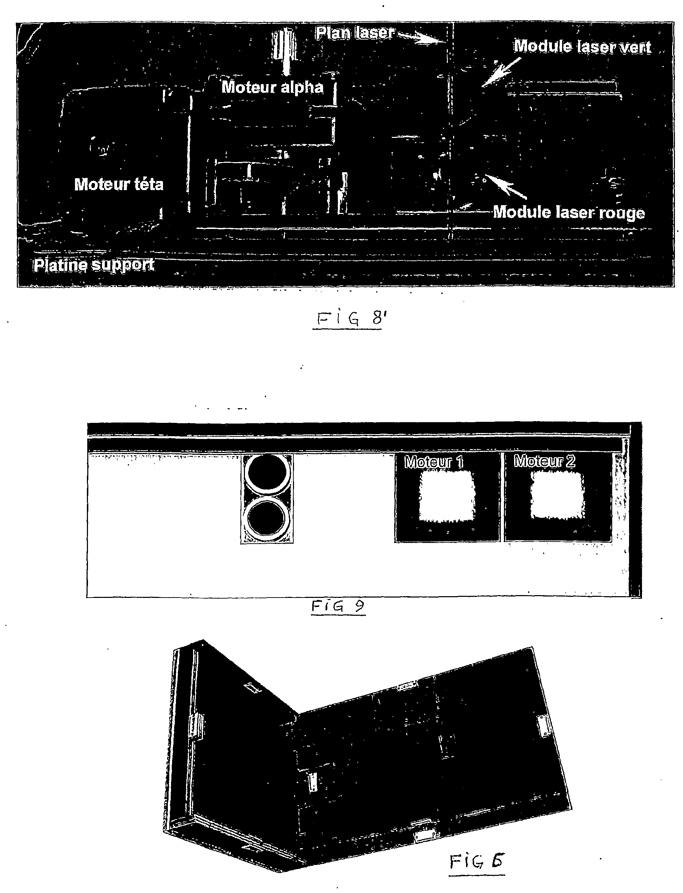

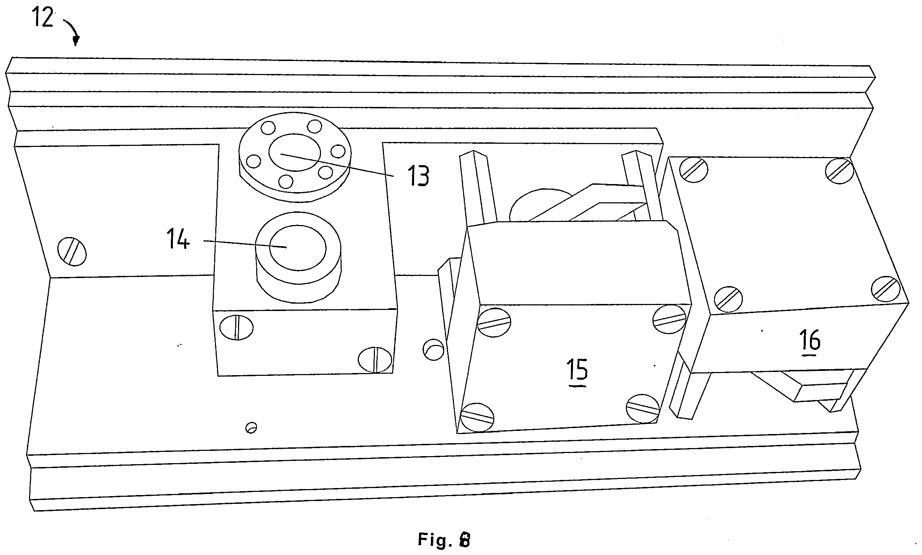

Des vues supplémentaires sont présentées sur les figures 8 et suivantes. Une fois les positions des nappes lasers 4 acquises par multiplexage, la carte de gestion 8 envoie ses informations au Tablet PC. Celui-ci donne ensuite ses ordres de réglages aux différentes têtes optiques 12 composant le système.

Le parfait positionnement des nappes 4 est contrôlé par l'appareil précité 1 dit fantôme intelligent. Le patient est placé sur la table du scanner. Les lasers mobiles sont sous tension et placés en leur point zéro. Un premier marquage à la peau est effectué par les manipulateurs. Une fois marqué, le patient est placé au centre du tunnel du scanner et des acquisitions d'images sont effectuées. La tumeur est alors localisée par le radiologue et apparaît sur la console de simulation virtuelle (TPS). Le médecin va alors détourer la tumeur, déterminant donc les valeurs exactes de position de la tumeur en trois dimensions. Ces informations sont transmises ensuite informatiquement à la console de pilotage des lasers mobiles.

Entre-temps, le patient est replacé à son point de départ et donc à nouveau positionné sur le point initial constituant le « 0 » des nappes lasers 4.

Les lasers mobiles se déplacent grâce à ces informations, et se placent à l'isocentre exact de la tumeur. Ils indiquent donc une nouvelle croix. C'est cette croix finale qui sera tatouée sur le patient par le manipulateur et qui servira de référence de position dans la salle de traitement.

L'invention porte également sur un dispositif optique comprenant un rail et une tête, et sur un outil dédié au réglage automatique du système constituant le dispositif intelligent, les têtes optiques 12 faisant également l'objet de l'invention. Le dispositif optique est composé des éléments suivants comme également illustré sur la figure 9. Un rail motorisé avec mesure de position précise au centième de millimètre et une tête optique 12 composée de moteurs ultra-précis 15 et 16 pour les mouvements suivant tous les degrés de liberté, des éléments mécaniques passifs pour le support des générateurs lasers rouge 13 et vert 14 et des éléments mécaniques passifs de support et d'articulation de l'ensemble.

Ainsi, cet appareil à tête optique 12 est constitué d'un ensemble mécanique à bras de levier, de deux moteurs pas-à-pas de précision équipés de capteurs de fin de course, de deux modules laser 13 et 14 et d'une carte électronique de commande.

Deux modules lasers génèrent une ligne de couleur rouge 13 et une ligne de couleur verte 14. Ces deux lignes 13 et 14 sont confondues dans un même plan. La fonctionnalité de la tête optique 12 est de pouvoir orienter ce plan sur deux axes différents α et θ représentés sur la figure 10. La valeur des angles est de +/-2° avec une précision de 10'.

Une carte électronique qui est pilotée par une liaison radio ZigBee, reçoit les informations du CPU central et commande les deux moteurs pas-à-pas 15 et 16.

En outre, il est également prévu un logiciel de pilotage qui pilote de façon très précise et sécurisée l'ensemble des systèmes installés sur les sites clients dont la présentation générale est exposée ci-après à titre d'exemple.

### Exemple

Ce logiciel est exploité dans un environnement réseau Microsoft Windows sur des ordinateurs de type PC de bureau et Tablet PC. Le logiciel est développé sous Microsoft Visual Basic (Studio 2010) et exploite une Base de Donnée de type SQL. Toutes les fonctionnalités dudit logiciel sont accessibles via un menu de type déroulant.

La figure 11 montre un schéma-bloc partiel d'un détail des fonctionnalités du logiciel, utilisé en tout ou en partie, éventuellement dans un ordre différent, est répertorié ci-après :
a) Paramétrage du logiciel : ce module permet de gérer tous les paramètres d'exploitation des données du logiciel.
b) Gestion des Utilisateurs : ce module permet de gérer la création, les mises à jour, la suppression et l'impression des données propres aux utilisateurs du programme. Chaque utilisateur est doté d'un niveau d'accès au programme.
c) Journalisation Système : l'utilisation de chacune des fonctionnalités ainsi que toutes les alarmes détectées font l'objet d'un enregistrement dans un journal.
d) Aide en Ligne : le logiciel est doté d'un « Guide de l'utilisateur » contextuel (touche F1) qui peut être consulté à tout moment sur l'écran du Tablet PC ou du PC de bureau. Ce « Guide de l'utilisateur » détaille toutes les fonctionnalités du logiciel. Tout ou une partie de ce « Guide de l'utilisateur » peut être imprimée sur demande de l'utilisateur.
e) Gestion des Alarmes : en cas de disfonctionnement de l'un ou l'autre des composants du système et détecté par le logiciel, ce dernier passe en mode « Défaut du Système ». Une liste déroulante des défauts répertoriés s'affiche à l'écran. En regard de chaque défaut, des instructions guident l'utilisateur dans la marche à suivre.
f) Sauvegarde/Restauration des Données d'Exploitation : ce module permet de sauvegarder automatiquement, ou sur demande de l'utilisateur, la totalité de la base de données du programme. Cette sauvegarde peut être effectuée, au choix de l'utilisateur, sur un support externe (clé USB, CD-ROM) ou sur un disque réseau. La restauration de la totalité de base de données est réalisée sur demande de l'utilisateur. Ce dernier a la possibilité de choisir le fichier de sauvegarde à utiliser pour la restauration.
g) Gestion des Patients et des Séances : le logiciel permet
   - de créer manuellement par saisie plusieurs patients, plusieurs champs (tumeurs) pour chacun de ces patients, plusieurs points de positionnement pour chacun de ces champs, et
   - de créer automatiquement les patients, champs et points de positionnement à partir des données importées depuis les fichiers TPS au format texte.
   L'objet d'une séance de traitement est de positionner les nappes lasers conformément aux consignes TPS (points de positionnement) en vue de procéder au marquage à la peau du patient. Le logiciel permet donc, dans le cadre de séances de traitement d'un patient, de
   - sélectionner un patient dans la Base de données,
   - sélectionner pour ce patient un champ (tumeur) dans la Base de données, et
   - sélectionner pour ce champ un ou plusieurs points de positionnement dans la Base de données.
   Si plusieurs points de positionnement sont sélectionnés par l'opérateur, ce dernier est invité à choisir un séquençage manuel ou un séquençage temporisé du pilotage des nappes laser. Dans le cas du manuel, le passage d'un point de positionnement au suivant est activé par l'opérateur qui est matérialisé par un clic sur bouton « Point suivant » à l'écran. Dans le cas du temporisé, la valeur de temporisation pour le passage d'un point de positionnement au suivant est renseignée par l'opérateur en début de séance.
   Pour chaque point de positionnement précédemment sélectionné par l'opérateur :
   le programme affiche à l'écran les informations propres au point de positionnement (coordonnées, désignation, etc.),
   l'opérateur est invité à autoriser (clic sur bouton à l'écran) le pilotage des nappes laser par le programme,
   le programme pilote les nappes laser compte tenu des valeurs de consignes de positionnement,
   le marquage à la peau est réalisé manuellement par l'opérateur,
   l'opérateur est invité à valider l'opération (clic sur bouton à l'écran),
   l'opération est enregistrée dans un historique des séances de traitement des patients.
h) Récupération des Données TPS : lors de chaque passage du patient dans le Scanner, l'opérateur procède au détourage par points de la tumeur. Ainsi, un fichier est généré par le système informatique de la console TPS. Ce fichier normalisé DICOM RT contient de nombreuses données, notamment en-tête données Scanner; données patient ; champs (tumeurs) et coordonnées des points cibles de chaque Champ pour le traitement en Radiothérapie.
   Le logiciel récupère les données ci-dessus et les intègre dans sa propre Base de Données.
i) Consultation/Impression des Données d'Exploitation : toutes les informations relatives aux données d'exploitation peuvent être consultées sur l'écran du Tablet PC ou du PC de bureau.
j) Gestion des Équipements : le logiciel permet de gérer la création, les mises à jour et l'impression de toutes les données de configuration des équipements qui composent le système de l'invention sur chaque site d'exploitation.
k) Autotest des Équipements : au démarrage du logiciel et après authentification de l'utilisateur, un autotest est réalisé par le programme pour vérifier l'intégrité du système. Cet autotest peut être lancé par ailleurs sur demande de l'utilisateur.
l) Sélection des Couleurs des Nappes Lasers : le Logiciel permet de sélectionner à tout instant et dans n'importe lequel des modules du programme la couleur (vert, rouge ou aucune) de chacune des nappes laser, individuellement ou pour l'ensemble des nappes.
m) Calibration manuelle des Équipements : la calibration manuelle de chacune des nappes laser (HIT Motorisés et HIT Mobiles) permet de régler et de calibrer le système lors de son installation, et ce compte tenu des caractéristiques physiques du site d'implantation. Cette opération peut également être réalisée en maintenance par un technicien compétent ou par une opératrice lorsqu'un contrôle des réglages et de la calibration est souhaité.
   Le logiciel enregistre dans un historique toutes les modifications apportées à la calibration de n'importe laquelle des nappes laser.
n) Calibration automatique des Équipements : la calibration automatique de chacune des nappes laser (HIT Motorisés et HIT Mobiles) est réalisée grâce à l'utilisation de l'équipement précité nommé Fantôme intelligent. Cet équipement est un système intelligent doté de capteurs (photorécepteurs) qui enregistrent la position de chacune des nappes en service. Le Fantôme est donc interrogé par le logiciel qui, compte tenu des informations collectées, pilote automatiquement les nappes laser selon les orientations souhaitées (coïncidence des nappes laser) et vers les positions recherchées (Isocentre). Le logiciel enregistre dans un historique toutes les modifications apportées à la calibration de n'importe laquelle des nappes laser.
o) Étalonnage des Axes : Le logiciel intègre la fonctionnalité d'étalonnage de chacune des nappes laser (HIT Motorisés et HIT Mobiles) exploitées dans le système. Cet étalonnage permet d'obtenir une précision de 0,1 mm de déplacement linéaire de chacune des nappes laser sur la peau du patient. Un étalonneur (capteur mobile) est utilisé pour procéder à l'étalonnage des axes.
p) Initialisation des Nappes lasers : le logiciel procède à une séquence d'initialisation laser dans les cas suivants : au démarrage du programme une fois l'autotest réalisé avec succès et/ou sur demande de l'utilisateur.
   Cette séquence d'initialisation effectue les opérations suivantes :
   Positionnement de chacune des têtes laser à l'origine des axes
   Positionnement de chacune des têtes laser compte tenu des données de calibration
   L'utilisateur sera informé de la bonne exécution de la séquence d'initialisation des nappes laser.
q) Consignes TPS : le logiciel est en mesure de positionner les nappes laser compte tenu de consignes liées à des points de positionnement sélectionnés par l'utilisateur.
   Ces points de positionnement correspondent aux points cibles issus des données TPS.
   Les nappes laser étant positionnées à l'Isocentre laser, ou ayant déjà fait l'objet d'une consigne TPS, ces nappes sont déplacées compte tenu des coordonnées indiquées par ces points de positionnement.
r) Placement du Patient : ce module permet de déplacer individuellement la nappe laser dite « Sagittale » vers la gauche ou vers la droite du patient en vue d'un marquage spécifique à la peau. Ce marquage à la peau ne correspond pas à un point de positionnement issu de données TPS.
   La valeur du déplacement de la nappe laser est renseignée (unité = cm) par l'opérateur. Plusieurs déplacements peuvent ainsi être effectués pour positionner la nappe « Sagittale » avant le marquage à la peau.
s) Consultation / impression des Données d'Équipements : toutes les informations relatives aux données d'équipements peuvent être consultées sur l'écran du Tablet PC ou du PC de bureau. L'édition de ces informations sur imprimante peut être demandée par l'utilisateur.

## Revendications

1. Procédé d'ajustement de nappes laser (4) pour scanners agencés sur un support, notamment dédiés à la radiothérapie, qui est disposé sur ledit support, au moyen d'un dispositif qui est constitué d'un ensemble unique monobloc (1) comprenant un certain nombre de pièces distinctes, et assemblé de façon indémontable, qui embarque à la fois un certain nombre de capteurs de position (3), et une carte électronique de gestion (8) adaptée à la structure monobloc dudit ensemble (1), lesdits capteurs de position (3) permettant de visualiser la position exacte desdites nappes (4), de façon à ajuster précisément celles-ci de manière automatique par des systèmes motorisés, **caractérisé en ce qu'**il comprend les étapes suivantes :
Mise en place du dispositif (1) sur la table du scanner ;
Préréglage de la position grâce aux trois pieds supports (6) et aux niveaux à bulles (7) embarqués ;
Déplacement de la table du scanner pour la mise en place de la face avant (11) de l'appareil dans le plan de l'isocentre du scanner et positionnement grâce aux lasers internes du scanner ;
Acquisition d'images, les images obtenues permettant de contrôler la position du dispositif (1) grâce à la présence d'au moins 4 billes de centrage (5) présentes sur la face avant (11) ; Finalisation de la position du dispositif ;
Une fois la position validée, déplacement de la table du scanner de la distance nominale entre l'isocentre machine et l'isocentre laser, cette distance étant fixe, qui est déterminée et validée lors de l'installation des lasers ;
Raccordement du dispositif (1) au moyen d'un Tablet PC gérant les positions des nappes lasers (4) ;
Mise sous tension du dispositif (1) ;
Mise sous tensions des nappes lasers (4) avec remise à 0 des positions ;
démarrage de la séquence de recherche des nappes lasers (4) par multiplexage, la position de chaque nappe laser (4) étant alors déterminée grâce aux capteurs de position embarqués (3) dans le dispositif (1) ;
acquisition des valeurs et transfert vers le Tablet PC de la phase d'ajustage de chaque nappe laser (4).

2. Procédé selon la revendication précédente, **caractérisé en ce que** ladite procédure d'utilisation comprend l'ensemble des étapes précitées effectuées dans l'ordre indiqué.

3. Dispositif d'ajustement de nappes laser (4) pour scanners agencés sur un support, notamment dédiés à la radiothérapie, en particulier pour la mise en oeuvre du procédé tel que défini dans l'une des revendications précédentes, **caractérisé en ce qu'**il est constitué d'un ensemble unique monobloc (1) comprenant un certain nombre de pièces distinctes, et assemblé de façon indémontable, **en ce qu'**il embarque comme pièces distinctes à la fois un certain nombre de capteurs de position (3) et une carte électronique de gestion (8) adaptée à la structure monobloc dudit ensemble (1), lesdits capteurs (3) étant agencés pour visualiser la position exacte desdites nappes laser (4), celles-ci (4) étant ajustées précisément via ladite carte électronique de gestion (8) utilisant lesdits capteurs (3) et ce de manière automatique par des systèmes motorisés; le dispositif étant équipé sur sa base (9) de trois pieds réglables (6) permettant un réglage en azimut dudit ensemble (1) sur la table du scanner, celui-ci comprenant des niveaux à bulles (7) qui sont disposés sur la plaque de base (9) pour aider au positionnement par des opérateurs et un certain nombre de billes de centrage (5) présentes sur la face avant (11).

4. Dispositif selon la revendication précédente, **caractérisé en ce qu'**il comprend deux éléments laser (13, 14) générant une nappe de couleur différente sélectionnable, en particulier rouge ou verte, au choix de l'opérateur.

5. Dispositif selon la revendication précédente, **caractérisé en ce que** ladite couleur de nappe laser est interchangeable instantanément.

6. Dispositif suivant l'une des revendications 3 à 5, **caractérisé en ce que** l'ensemble monobloc précité (1) comprend sept pièces distinctes, et **en ce qu'**il est prévu au moins dix, plus particulièrement douze cellules capteurs de position (3).

7. Dispositif selon l'une des revendications 3 à 6, **caractérisé en ce qu'**il s'agit d'un dispositif portable (1) et/ou **en ce qu'**il incorpore deux éléments laser industriel standard.

8. Dispositif selon l'une des revendications 3 à 7, **caractérisé en ce qu'**il inclut un logiciel dédié et adapté aux contraintes d'une utilisation en milieu hospitalier.

9. Dispositif selon la revendication précédente, **caractérisé en ce que** les capteurs de position sont des cellules de mesure intégrées (3) avec dimension calculée pour la meilleure précision, étant dédié au réglage automatique du système, de façon à permettre une mesure précise de la position de chaque nappe précitée (4) et un réglage de chaque nappe l'une par rapport à l'autre, ainsi qu'un réglage en boucle fermée à partir dudit logiciel.

10. Dispositif selon l'une des revendications 5 à 9, **caractérisé par** un positionnement garanti avec une précision finale d'au moins 0,1 mm.

11. Dispositif d'ajustement de nappes laser pour scanners sur support, dédiés à la radiothérapie, en particulier selon l'une des revendications 3 à 10, **caractérisé en ce qu'**il comprend au moins une tête optique (12) mécanisée avec des éléments mécaniques de précision calibrés pour garantir les degrés de liberté nécessaire, particulièrement **en ce qu'**il comprend des moyens de filtrage des perturbations vibratoires externes, plus particulièrement **en ce qu'**il comprend des moyens de guidage, en particulier un rail pour les mouvements desdites têtes optiques (12) avec roulements mécaniques de précision intégrant une mesure de position de la tête optique (12) sur son rail.

12. Dispositif selon la revendication précédente, **caractérisé en ce qu'**il comprend un ensemble mécanique à bras de levier, constitué de deux moteurs pas-à-pas de précision (15, 16) équipés de capteurs de fin de course, de deux modules laser (13, 14) et d'une carte électronique de commande, pouvant être pilotée par une liaison radio, recevoir les informations du processeur central et commander les deux moteurs pas-à-pas précités (15, 16).

13. Dispositif selon l'une des revendications 3 à 12, **caractérisé en ce qu'**il comprend des moyens de vérification de la position exacte des nappes laser (4) en fonction des consignes prévues pour être données ou à fournir par l'opérateur.

14. Procédé selon l'une des revendications 1 ou 2 utilisant un logiciel pour la mise en oeuvre d'au moins une partie des étapes resp. phases du procédé, notamment au moyen du dispositif selon l'une des revendications 3 à 13, **caractérisée par** des modules constituant les fonctionnalités du logiciel utilisé en tout ou en partie, éventuellement dans un ordre différent, qui sont répertoriées ci-après :
- Paramétrage (a) du logiciel permettant de gérer tous les paramètres d'exploitation des données du logiciel ;
- Gestion (b) des Utilisateurs permettant de gérer la création, les mises à jour, la suppression et l'impression des données propres aux utilisateurs du programme, chaque utilisateur étant doté d'un niveau d'accès au programme ;
- Journalisation Système (c), l'utilisation de chacune des fonctionnalités ainsi que toutes les alarmes détectées faisant l'objet d'un enregistrement dans un journal ;
- Aide en Ligne (d), le logiciel étant doté d'un Guide d'utilisateur contextuel, qui peut être consulté à tout moment sur l'écran du Tablet PC ou du PC de bureau et qui détaille toutes les fonctionnalités du logiciel ;
- Gestion des Alarmes (e), le logiciel passant en mode « Défaut du Système » en cas de dysfonctionnement de l'un ou l'autre des composants du système et détecté par ce dernier, une liste déroulante des défauts répertoriés s'affichant à l'écran, et des instructions guidant l'utilisateur dans la marche à suivre en regard de chaque défaut ;
- Sauvegarde/Restauration (f) des Données d'Exploitation permettant de sauvegarder automatiquement, ou sur demande, la totalité de la base de données du programme ;
- Gestion des Patients et des Séances (g) permettant de créer manuellement par saisie plusieurs patients, plusieurs champs notamment tumoral pour chacun de ces patients, plusieurs points de positionnement pour chacun de ces champs, ainsi que de créer automatiquement les patients, champs et points de positionnement à partir des données importées depuis les fichiers TPS au format texte ;
- Récupération des Données TPS (h), l'opérateur procédant au détourage par points de la tumeur lors de chaque passage du patient dans le Scanner, un fichier étant ainsi généré par le système informatique de la console TPS, le logiciel récupérant les données pour les intégrer dans sa propre Base de Données ;
- Consultation/Impression (i) des Données d'Exploitation, toutes les informations relatives aux données d'exploitation pouvant être consultées sur écran ;
- Gestion des Équipements (j), le logiciel permettant de gérer la création, les mises à jour et l'impression de toutes les données de configuration des équipements qui composent le système de l'invention sur chaque site d'exploitation ;
- Autotest des Équipements (k), un autotest étant réalisé par le programme au démarrage du logiciel et après authentification de l'utilisateur pour vérifier l'intégrité du système, qui peut être lancé par ailleurs sur demande de l'utilisateur ;
- Sélection des Couleurs des Nappes Lasers (I), le logiciel permettant de sélectionner à tout instant et dans n'importe lequel des modules du programme la couleur (vert, rouge ou aucune) de chacune des nappes laser, individuellement ou pour l'ensemble des nappes ;
- Calibration manuelle des Équipements (m), la calibration manuelle de chacune des nappes laser (HIT Motorisés et HIT Mobiles) permettant de régler et de calibrer le système lors de son installation, compte tenu des caractéristiques physiques du site d'implantation ;
- Calibration automatique (n) des Équipements, la calibration automatique de chacune des nappes laser -HIT Motorisés et HIT Mobiles- étant réalisée par l'utilisation de l'équipement précité consistant en un système intelligent doté de capteurs ou photorécepteurs qui enregistrent la position de chacune des nappes en service, le système précité étant interrogé par le logiciel qui, compte tenu des informations collectées, pilote automatiquement les nappes laser selon les orientations souhaitées par coïncidence des nappes laser, et vers les positions recherchées en Isocentre, le logiciel enregistrant dans un historique toutes les modifications apportées à la calibration de n'importe laquelle des nappes laser ;
- Étalonnage des Axes (o), le logiciel intégrant la fonctionnalité d'étalonnage de chacune des nappes laser -HIT Motorisés et HIT Mobiles- exploitées dans le système, cet étalonnage permettant d'obtenir une précision allant jusqu'à au moins 0,1 mm de déplacement linéaire de chacune des nappes laser sur la peau du patient, un étalonneur ou capteur mobile étant utilisé pour procéder à l'étalonnage des axes ;
- Initialisation des Nappes lasers (p), le logiciel procédant à une séquence d'initialisation laser dans certains cas au démarrage du programme une fois l'autotest réalisé avec succès et/ou sur demande de l'utilisateur, ladite séquence d'initialisation effectuant les opérations de positionnement de chacune des têtes laser à l'origine des axes, resp. Positionnement de chacune des têtes laser compte tenu des données de calibration, l'utilisateur étant informé de la bonne exécution de la séquence d'initialisation des nappes laser ;
- Consignes TPS (q), le logiciel pouvant positionner les nappes laser compte tenu de consignes liées à des points de positionnement sélectionnés par l'utilisateur, ces points de positionnement correspondant aux points cibles issus des données TPS ; les nappes laser étant positionnées à l'Isocentre laser, ou ayant déjà fait l'objet d'une consigne TPS, étant déplacées compte tenu des coordonnées indiquées par ces points de positionnement ;
- Placement du Patient (r) permettant de déplacer individuellement la nappe laser dite Sagittale vers la gauche ou vers la droite du patient en vue d'un marquage spécifique à la peau, la valeur du déplacement de la nappe laser étant renseignée par l'opérateur ;
- Consultation/impression (s) des Données d'Équipements.

15. Procédé selon la revendication précédente, **caractérisé par** l'utilisation de l'ensemble des étapes effectuées selon le logiciel précité, en particulier selon l'ordre indiqué et/ou en ce que l'édition de chacune ou partie de ces informations sur imprimante est sélectionnable par l'utilisateur.

## Patentansprüche

1. Verfahren zur Justierung von Laser-Lichtwänden (4) für Scanner, die auf einem Träger angeordnet sind, die insbesondere für die Strahlentherapie bestimmt sind, die auf diesem Träger angeordnet ist, mit Hilfe einer Vorrichtung, die von einer einstückigen Einheit (1) gebildet ist, die eine gewisse Anzahl von unterschiedlichen Teilen umfasst und unlösbar zusammengefügt ist, die sowohl eine gewisse Anzahl an Positionssensoren (3) als auch eine elektronische Steuerkarte (8) umfasst, die an die einstückige Struktur der Einheit (1) angepasst ist, wobei es die Positionssensoren (3) ermöglichen, die exakte Position der Lichtwände (4) zu visualisieren, um diese präzise automatisch durch motorbetriebene Systeme zu justieren, **dadurch gekennzeichnet, dass** es die folgenden Schritte umfasst:
Anbringen der Vorrichtung (1) auf dem Tisch des Scanners;
Voreinstellen der Position mit Hilfe der drei Stützfüße (6) und der eingebauten Wasserwagen (7);
Verschieben des Tisches des Scanners zum Anordnen der Vorderseite (11) des Geräts in der Ebene des Isozentrums des Scanners und Positionierung dank der internen Laser des Scanners;
Bilderfassung, wobei es die erhaltenen Bilder ermöglichen, die Position der Vorrichtung (1) dank des Vorhandenseins von mindestens 4 Zentrierkugeln (5), die auf der Vorderseite (11) vorgesehen sind, zu kontrollieren;
Finalisieren der Position der Vorrichtung;
wenn die Position validiert ist, Verschieben des Tisches des Scanners um die Nominaldistanz zwischen dem Isozentrum der Maschine und dem Isozentrum des Lasers, wobei diese Distanz feststehend ist und bei der Installation des Lasers bestimmt und validiert wird;
Anschluss der Vorrichtung (1) mit Hilfe eines Tablet PC, der die Positionen der Laser-Lichtwände (4) steuern;
Einschalten der Vorrichtung (1);
Einschalten der Laser-Lichtwände (4) mit Rücksetzung der Positionen;
Starten der Suchsequenz der Laser-Lichtwände (4) durch Multiplexing, wobei die Position jeder Laser-Lichtwand (4) nun mit Hilfe der in die Vorrichtung (1) eingebauten Positionssensoren (3) bestimmt wird;
Erfassung der Werte und Weitergabe der Justierphase jeder Laser-Lichtwand (4) an den Tablet PC.

2. Verfahren nach dem vorhergehenden Anspruch, **dadurch gekennzeichnet, dass** der Verwendungsvorgang die Gesamtheit der vorgenannten Schritte, ausgeführt in der angegebenen Reihenfolge, umfasst.

3. Vorrichtung zur Justierung von Laser-Lichtwänden (4) für Scanner, die auf einem Träger angeordnet sind, die insbesondere für die Strahlentherapie bestimmt sind, insbesondere für den Einsatz des Verfahrens, wie in einem der vorhergehenden Ansprüche definiert, **dadurch gekennzeichnet, dass** sie von einer einstückigen Einheit (1) gebildet ist, die eine gewisse Anzahl von unterschiedlichen Teilen umfasst und unlösbar zusammengefügt ist, dass sie als unterschiedliche Teile sowohl eine gewisse Anzahl an Positionssensoren (3) und eine elektronische Steuerkarte (8) umfasst, die an die einstückige Struktur der Einheit (1) angepasst ist, wobei die Sensoren (3) derart angeordnet sind, dass sie die exakte Position der Laser-Lichtwände (4) visualisieren, wobei diese (4) präzise über die elektronische Steuerkarte (8) unter Verwendung der Sensoren (3) justiert werden, und zwar automatisch durch motorbetriebene Systeme; wobei die Vorrichtung an ihrer Basis (9) mit drei einstellbaren Füßen (6) ausgestattet ist, die eine Azimut-Einstellung der Einheit (1) auf dem Tisch des Scanners ermöglichen, wobei dieser letztgenannte Wasserwaagen (7), die auf der Grundplatte (9) angeordnet sind, um bei der Positionierung durch Bediener zu helfen, und eine gewisse Anzahl von Zentrierkugeln (5), die auf der Vorderseite (11) vorhanden sind, umfasst.

4. Vorrichtung nach dem vorhergehenden Anspruch, **dadurch gekennzeichnet, dass** sie zwei Laserelemente (13, 14) umfasst, die eine Lichtwand von unterschiedlicher wählbarer Farbe, insbesondere rot oder grün, nach Wunsch des Bedieners erzeugt.

5. Vorrichtung nach dem vorhergehenden Anspruch, **dadurch gekennzeichnet, dass** die Farbe der Laser-Lichtwand augenblicklich austauschbar ist.

6. Vorrichtung nach einem der Ansprüche 3 bis 5, **dadurch gekennzeichnet, dass** die vorgenannte einstückige Einheit (1) sieben unterschiedliche Teile umfasst, und dass mindestens zehn, insbesondere zwölf, Positionssensorzellen (3) vorgesehen sind.

7. Vorrichtung nach einem der Ansprüche 3 bis 6, **dadurch gekennzeichnet, dass** es sich um eine tragbare Vorrichtung (1) handelt, und/oder dass sie zwei Standard-Industrielaserelemente einschließt.

8. Vorrichtung nach einem der Ansprüche 3 bis 7, **dadurch gekennzeichnet, dass** sie eine spezielle Software einschließt, die an die Anforderungen einer Verwendung im Spitalsumfeld angepasst ist.

9. Vorrichtung nach dem vorhergehenden Anspruch, **dadurch gekennzeichnet, dass** die Positionssensoren integrierte Messzellen (3) mit einer für die beste Präzision berechneten Dimension, speziell für die automatische Einstellung des Systems, sind, um eine präzise Messung der Position jeder vorgenannten Lichtwand (4) und eine Einstellung jeder Lichtwand in Bezug zueinander sowie eine Einstellung in geschlossener Schleife auf Basis der Software zu ermöglichen.

10. Vorrichtung nach einem der Ansprüche 5 bis 9, **gekennzeichnet durch** eine garantierte Positionierung mit einer finalen Präzision von mindestens 0,1 mm.

11. Vorrichtung zur Justierung von Laser-Lichtwänden für Scanner auf einem Träger, die für die Strahlentherapie bestimmt sind, insbesondere nach einem der Ansprüche 3 bis 10, **dadurch gekennzeichnet, dass** sie mindestens einen optischen Kopf (12) umfasst, der mit kalibrierten mechanischen Präzisionselementen mechanisiert ist, um die notwendigen Freiheitsgrade zu garantieren, insbesondere dass sie Mittel zur Filterung der externen Schwingungsstörungen umfasst, insbesondere dass sie Führungsmittel umfasst, insbesondere eine Schiene für die Bewegungen der optischen Köpfe (12) mit mechanischen Präzisionslagern, einschließlich einer Positionsmessung des optischen Kopfes (12) auf seiner Schiene.

12. Vorrichtung nach dem vorhergehenden Anspruch, **dadurch gekennzeichnet, dass** sie eine mechanische Einheit mit Hebelarm umfasst, die von zwei Schritt-Präzisionsmotoren (15, 16) gebildet ist, die mit Endanschlagsensoren, zwei Lasermodulen (13, 14) und einer elektronischen Steuerkarte ausgestattet sind, die über eine Funkverbindung gesteuert werden kann, die Informationen von dem Zentralprozessor erhalten und die zwei vorgenannten Schrittmotoren (15, 16) steuern kann.

13. Vorrichtung nach einem der Ansprüche 3 bis 12, **dadurch gekennzeichnet, dass** sie Mittel zur Überprüfung der exakten Position der Laser-Lichtwände (4) in Abhängigkeit von den vorgesehenen Sollwerten, die vom Bediener vorzugeben oder zu liefern sind, umfasst.

14. Verfahren nach einem der Ansprüche 1 oder 2, das eine Software für den Einsatz mindestens eines Teils der Schritte bzw. Phasen des Verfahrens insbesondere mit Hilfe der Vorrichtung nach einem der Ansprüche 3 bis 13 verwendet, **gekennzeichnet durch** Module, die die Funktionalitäten der verwendeten Software zur Gänze oder teilweise eventuell in einer unterschiedlichen Reihenfolge, die nachstehend angeführt sind, darstellen:
- Parametrierung (a) der Software, die es ermöglicht, alle Betriebsparameter der Daten der Software zu steuern;
- Steuerung (b) der Benutzer, die es ermöglicht, die Erzeugung, Aktualisierung, das Löschen und das Drucken der den Benutzern des Programms eigenen Daten zu steuern, wobei jeder Benutzer mit einem Zugriffslevel auf das Programm versehen ist;
- System-Protokollierung (c), wobei die Verwendung jeder der Funktionalitäten sowie aller erfassten Alarme Gegenstand einer Registrierung im Protokoll sind;
- Online-Hilfe (d), wobei die Software mit einem kontextabhängigen Benutzerhandbuch versehen ist, das jederzeit auf dem Bildschirm des Tablet PC oder des Desktop-PC konsultiert werden kann, und das alle Funktionalitäten der Software im Detail anführt;
- Steuerung der Alarme (e), wobei die Software in den Modus "Systemfehler" im Falle einer Fehlfunktion der einen oder der anderen der Komponenten des Systems übergeht, die von dieser letztgenannten erfasst wird, wobei eine ablaufende Fehlerliste und Anweisungen, die den Benutzer bei der Vorgehensweise im Hinblick auf jeden Fehler leiten, auf dem Bildschirm angezeigt werden;
- Speichern/Wiederherstellen (f) der Betriebsdaten, das es ermöglicht, automatisch oder auf Befehl die Gesamtheit der Datenbasis des Programms zu speichern;
- Verwaltung der Patienten und Sitzungen (g), die es ermöglicht, manuell durch Erfassung mehrerer Patienten mehrere insbesondere Tumorfelder für jeden dieser Patienten, mehrere Positionierungspunkte für jedes dieser Felder zu erzeugen, sowie automatisch die Patienten, Felder und Positionierungspunkte auf Basis der von den TPS-Dateien im Textformat importierten Daten zu erzeugen;
- Wiederverwertung der TPS-Daten (h), wobei der Bediener den Tumor bei jedem Einbringen des Patienten in den Scanner mit Punkten zuschneidet, wobei auf diese Weise eine Datei von dem Informatiksystem der TPS-Konsole erzeugt wird, wobei die Software die Daten wiederverwertet, um sie in ihre eigene Datenbasis zu integrieren;
- Konsultation/Drucken (i) der Betriebsdaten, wobei alle Informationen in Zusammenhang mit den Betriebsdaten auf dem Bildschirm eingesehen werden können;
- Steuerung der Ausrüstungen (j), wobei es die Software ermöglicht, die Erzeugung, Aktualisierungen und das Drucken aller Konfigurationsdaten der Ausrüstungen, aus denen das erfindungsgemäße System an jedem Betriebsstandpunkt besteht, zu steuern;
- Autotest der Ausrüstungen (k), wobei ein Autotest von dem Programm beim Starten der Software und nach Authentifizierung des Benutzers durchgeführt wird, um die Integrität des Systems zu überprüfen, der überdies auf Befehl des Benutzers gestartet werden kann;
- Auswahl der Farben der Laser-Lichtwände (1), wobei es die Software ermöglicht, jederzeit und in jedem der Module des Programms die Farbe (grün, rot oder keine) jeder der Laser-Lichtwände individuell oder für die Gesamtheit der Lichtwände auszuwählen;
- manuelle Kalibrierung der Ausrüstungen (m), wobei es die manuelle Eichung jeder der Laser-Lichtwände (HIT motorisiert und HIT mobil) ermöglicht, das System bei seiner Installation unter Berücksichtigung der physikalischen Eigenschaften des Aufstellungsstandortes zu einzustellen und zu kalibrieren;
- automatische Kalibrierung (n) der Ausrüstungen, wobei die automatische Kalibrierung jeder der Laser-Lichtwände - HIT motorisiert und HIT mobil - durch die Verwendung der vorgenannten Ausrüstung durchgeführt wird, die in einem intelligenten System besteht, das mit Sensoren oder Fotorezeptoren versehen ist, die die Position jeder der Lichtwände in Betrieb aufzeichnen, wobei das vorgenannte System von der Software befragt wird, die auf Grund der gesammelten Informationen automatisch die Laser-Lichtwände in die gewünschten Richtungen durch Koinzidenz der Laser-Lichtwände und in die angestrebten Positionen im Isozentrum steuert, wobei die Software in einer Historie alle Änderungen aufzeichnet, die an der Kalibrierung einer beliebigen Laser-Lichtwand vorgenommen wurden;
- Eichung der Achsen (o), wobei die Software die Eichungsfunktionalität jeder der Laser-Lichtwände - HIT motorisiert und HIT mobil -, die in dem System genutzt werden, integriert, wobei es diese Eichung ermöglicht, eine Präzision zu erzielen, die bis zu mindestens 0,1 mm Linearverschiebung jeder der Laser-Lichtwände auf der Haut des Patienten geht, wobei ein mobiles Eichungselement oder ein mobiler Sensor verwendet wird, um die Eichung der Achsen vorzunehmen;
- Initialisierung der Laser-Lichtwände (p), wobei die Software eine Laser-Initialisierungssequenz in gewissen Fällen beim Starten des Programms durchführt, wenn der Autotest erfolgreich und/oder auf Befehl des Benutzers durchgeführt wurde, wobei die Initialisierungssequenz die Vorgänge einer Positionierung jedes der Laserköpfe am Ursprung der Achsen bzw. einer Positionierung jedes der Laserköpfe unter Berücksichtigung der Kalibrierungsdaten durchführt, wobei der Benutzer über die ordnungsgemäße Ausführung der Initialisierungssequenz der Laser-Lichtwände informiert wird;
- TPS-Sollwerte (q), wobei die Software die Laser-Lichtwände unter Berücksichtigung von Sollwerten in Verbindung mit vom Benutzer ausgewählten Positionierungspunkten positionieren kann, wobei diese Positionierungspunkte den Zielpunkten, die aus den TPS-Daten hervorgehen, entsprechen; wobei die Laser-Lichtwände im Laser-Isozentrum positioniert werden, oder wenn sie bereits Gegenstand eines TPS-Sollwerts waren, unter Berücksichtigung der durch diese Positionierungspunkte angezeigten Koordinaten verschoben werden;
- Platzieren des Patienten (r), das es ermöglicht, die so genannte Sagittal-Laser-Lichtwand individuell zur linken oder zur rechten Seite des Patienten für eine spezifische Markierung auf der Haut zu verschieben, wobei der Wert der Verschiebung der Laser-Lichtwand vom Bediener mitgeteilt wird;
- Konsultation/Drucken (s) der Ausrüstungsdaten.

15. Verfahren nach dem vorhergehenden Anspruch, **gekennzeichnet durch** die Verwendung der Gesamtheit der Schritt, die gemäß der vorgenannten Software, insbesondere in der angegebenen Reihenfolge, ausgeführt werden, und/oder dass die Ausgabe jeder oder eines Teils dieser Informationen auf einem Drucker vom Benutzer wählbar ist.

## Claims

1. Method for adjusting laser beams (4) for scanners arranged on a support, notably dedicated to radiotherapy, which is positioned on said support, by means of a device which consists of a single one-piece assembly (1) comprising a certain number of distinct parts, and assembled in a manner in which it cannot be dismantled, which includes both a certain number of position sensors (3), and an electronic control board (8) suited to the one-piece structure of said assembly (1), said sensors (3) making it possible to visualize the exact position of said beams (4), so as to accurately adjust them automatically by motor-driven systems, **characterized in that** it comprises the following steps :
installing the apparatus (1) on the table of the scanner;
presetting the position using the three supporting feet (6) and the embedded spirit levels (7);
moving the table of the scanner to position the front face (11) of the apparatus in the plane of the isocenter of the scanner, and a positioning using the internal lasers of the scanner;
acquisition of images, wherein the images obtained make it possible to check the correct position of the apparatus (1) by using at least 4 centering balls (5) present on the front face (11);
finalizing the position of the apparatus;
once the position is validated, moving the table of the scanner by the nominal distance between the machine isocenter and the laser isocenter, wherein this distance is set, which is determined and validated at the time of installation of the lasers;
connecting the apparatus (1) to a PC tablet controlling the positions of the laser beam (4);
powering the apparatus (1) up,
powering the lasers beams (4) up and re-setting the positions to zero;
starting up the laser beam (4) search sequence by multiplexing, wherein the position of each laser beam (4) is then determined using the embedded cells (3) in the apparatus (1);
doing the acquisition of the values and transfer to the tablet PC of the adjustment phase of each laser beam (4).

2. Method according to the preceding claim, **characterized in that** said process of use comprises each of said steps being carried out in the specified order.

3. Device for adjusting laser beams (4) for scanners arranged on a support, notably dedicated to radiotherapy, which is positioned on said support, in particular for implementing the method as defined in one of the preceding claims, **characterized in that** it consists of a single one-piece assembly (1) comprising a certain number of distinct parts, and assembled in a manner in which it cannot be dismantled, **in that** it includes both a certain number of position sensors (3), and an electronic control board (8) suited to the one-piece structure of said assembly (1), said sensors (3) being arranged to visualize the exact position of said beams (4), so as to accurately adjust them automatically by motor-driven systems; the device being equipped on its base (9) with three adjustable feet (6) allowing for an azimuth adjustment of said assembly (1) on the table of the scanner, the latter comprising spirit levels (7) which are positioned on the base plate (9) to assist in the positioning by operators and a certain number of centering balls (5) present on the front face (11).

4. Device according to the preceding claim, **characterized in that** it comprises two laser elements (13, 14) generating a beam with a different color which is selectable, particularly red or green, up to the choice of the operator.

5. Device according to the preceding claim, **characterized in that** said color of the laser beam is instantly interchangeable.

6. Device according to one of the claims 3 to 5, **characterized in that** said single block set (1) comprises seven distinct parts, and wherein at least ten, more particularly twelve position sensors (3) are provided.

7. Device according to one of the claims 3 to 6, **characterized in that** it is a portable apparatus (1) and/or **in that** it incorporates two standard industrial laser elements.

8. Device according to one of the claims 3 to 7, **characterized in that** it includes dedicated software suited to the constraints of use in a hospital environment.

9. Device according to the preceding claim, **characterized in that** the position sensors consist of integrated measurement cells (3), with dimension calculated for better accuracy, being dedicated to an automatic adjustment of the system for allowing an accurate measurement of the position of each said beam (4), and adjustment of each beam relative to one another, as well as an adjustment in closed loop mode using said software.

10. Device according to one of the claims 5 to 9, **characterized in that** it has a guaranteed positioning result, with a final accuracy of at least 0,1 mm.

11. Device for adjusting laser beams for scanners on a support dedicated to radiotherapy, in particular according to one of the claims 3 to 10, **characterized in that** it has at least one mechanized optical head (12) with calibrated mechanical precision elements for ensuring the requested degrees of freedom, particularly wherein it comprises filtering means of the external vibratory disturbances, more particularly wherein it comprises guiding means, in particular a rail for the movements of said optical heads (12) with precision mechanical rolling bearings incorporating a measurement of the position of the optical head (12) on its rail.

12. Device according to the preceding claim, **characterized in that** it comprises a mechanical assembly with lever arm, made up of two precision stepper motors (15, 16) equipped with end-of-travel sensors, two laser modules (13, 14) and an electronic control board, which allows for being driven by a radio link, for receiving the information from the central CPU and for controlling the two said stepper motors (15, 16).

13. Device according to one of the claims 3 to 12, **characterized in that** it comprises controlling means for the verification of the exact position of the laser beams (4) according to the setpoints to be given or to provide by the operator.

14. Method according to one of the claims 1 or 2, using a software for carrying out at least part of the steps, resp. stages of the method, notably by means of the device according to one of the claims 3 to 13, wherein it has modules forming the functionalities of the software used wholly or partly, possibly in a different order, which are listed herein below:
- Parameterization (a) of the software allowing managing all the software data operating parameters;
- User management (b) allowing managing the creation, the updating, the deletion and printing of data specific to the program users, wherein each user is provided with a program access level;
- System logging (c), wherein the use of each of the functionalities and all the alarms detected are the subject of a record in a log;
- Online help (d), wherein the software is provided with a context-sensitive "User Guide", which can be viewed at any time on the screen of the tablet PC or on the desktop PC and which details all the functionalities of the software;
- Alarm management (e), wherein in the event of a malfunction of one or other of the components of the system and detected by the software, the latter switches to "System fault mode", wherein a pop-up list of the faults listed is displayed on the screen and, wherein alongside each fault, setpoints guide the user in what to do;
- Backup/restore (f) operating data, allowing to backup automatically, or at the request, all of the program database;
- Patient and session management (g) allowing creating manually by input a number of patients, a number of fields notably tumors for each of these patients, a number of positioning points for each of these fields, and automatically creating the patients, fields and positioning points from data imported from the text-format TPS files;
- TPS data recovery (h), wherein each time the patient passes through the scanner, the operator proceeds with outlining the tumor by dots, wherein a file is thus generated by the TPS console computer system, wherein the software recovers the above data and incorporates them in its own database;
- Display/print operating data (i), wherein all the information concerning the operating data can be displayed on the screen;
- Equipment management (j), wherein the software allows controlling the creation, the updating and the printing of all the configuration data of the equipment that make up the system of the invention on each operating site;
- Equipment self-test (k), wherein on starting up the software and after authentication of the user, a self-test is carried out by the program to check the integrity of the system, wherein said self-test can also be launched at the request of the user;
- Selection of the laser beam colors (I), wherein the software allows selecting, at any time and in any of the modules of the program, the color (green, red or none) of each of the laser beams, individually or for all of the beams;
- Manual equipment calibration (m), wherein the manual calibration of each of the laser beams (motor-driven HITs and moving HITs) allows adjusting and calibrating the system during its installation, and do so in light of the physical characteristics of the installation site;
- Automatic equipment calibration (n), wherein the automatic calibration of each of the laser beams -motor-driven HITs and moving HITs- is carried out with the use of the abovementioned so-called smart phantom equipment, wherein it is a smart system provided with sensors or photoreceivers which record the position of each of the beams in service, wherein said system is interrogated by the software which, given the information collected, automatically drives the laser beams according to the desired orientations by coincidence of the laser beams, and to the positions sought in isocenter, wherein the software keeps a historical log recording all the modifications made on calibrating any of the laser beams;
- calibration of the axes (o), wherein the software includes the calibration functionality for each of the laser beams -motor-driven HITs and moving HITs- operated in the system, wherein this calibration allows obtaining an accuracy of up to 0.1 mm of linear movement of each of the laser beams on the skin of the patient, wherein a calibrator or moving sensor is used to calibrate the axes;
- Laser beams initialization (p), wherein the software carries out a laser initialization sequence in the following cases: when the program is started up once the self-test has been successfully carried out and/or at the request of the user, wherein said initialization sequence performs the operations of positioning each of the laser heads at the origin of the axes, resp.
Positioning of each laser head in light of the calibration data, wherein the user is informed of the correct execution of the laser beams initialization sequence;
- TPS setpoints (q), wherein the software is able to position the laser beams in light of setpoints linked to positioning points selected by the user, wherein these positioning points correspond to the target points derived from the TPS data, wherein the laser beams are positioned at the laser isocenter, or already having been the object of a TPS setpoint, these beams are moved in light of the coordinates indicated by these positioning points;
- patient placement (r) allowing individually moving the so-called "sagittal" laser beam to the left or to the right of the patient for a specific marking on the skin, wherein the value of the movement of the laser beam is entered by the operator; and
- display/print (s) equipment data.

15. Use according to the preceding claim, **characterized in that** the process of use comprises each of the steps carried out according to said software, particularly in the specified order, and/or **in that** the output of all or part of this information on a printer can be requested by the user.
